# EUROPEAN PATENT APPLICATION

(11) **EP 2 343 296 A1**
(43) Date of publication of application: **13.07.2011**
(21) Application number: 09382266.6
(22) Date of filing: 01.12.2009
(51) Int. Cl.: C07D 471/04, A61K 31/517

(54) **A process for the purification of paliperidone**

(71) Applicant: Chemo Ibérica, S.A., 08028 Barcelona (ES)
(72) Inventor: Rasparini, MARCELLO, 27010 Cura Carpignano (PV) (IT); Ghisleri, DIEGO, 27050 Casei Gerola (PV) (IT)
(74) Representative: ZBM Patents

(57) **Abstract**

The process comprises submitting crude Paliperidone to an esterification reaction in the presence of a basic agent to yield a compound of formula (III), wherein n is 1-8.
This process gives Paliperidone with high purity after hydrolysis.

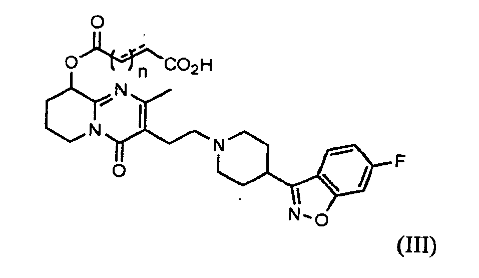

## Description

### Field of the invention

The present invention relates to a process for the purification of the antipsychotic agent Paliperidone and to intermediate thereof.

### Background of the invention

Paliperidone is a potent antagonist of neurotransmitters serotonin and dopamine. Antagonizing said mediators will suppress or relieve a variety of symptoms associated with phenomena induced by the excessive release of these mediators.

Serotonin antagonists are reportedly effective in combating psychoses, aggressive behaviour, anxiety, depression and migraine. Central acting serotonin antagonists appear to improve the negative symptoms of schizophrenia, e.g. anergy, apathy, social withdrawal and depressive mood, and also to reduce the incidence of extrapyramidal side-effects (EPS) during maintenance therapy with classical neuroleptics, i.e. dopamine antagonists.

Dopamine receptor antagonists are known to have neuroleptic properties, for example, they counteract the positive symptoms of schizophrenia, e.g. hallucinations, delusional thinking, severe excitement and unusual behaviour.

Combined serotonin-dopamine antagonists are especially interesting as they appear to offer relief of both the positive and negative symptoms of schizophrenia, with low EPS liability. Actually, these drugs display a decreased propensity to cause extrapyramidal side effects and an absence of sustained prolactin elevation.

Therapeutic indications for using Paliperidone therefore are mainly in the CNS area, particularly as potent antipsychotic agent and especially as agents useful in treating chronic psychoses, schizophrenia and bipolar disorders. Atypical antipsychotics, second-generation drugs, like Paliperidone, are now considered to be first line treatments for schizophrenia and are gradually replacing the typical antipsychotics.

Paliperidone also appear to be a useful therapeutic agent for combating autism. Therapeutic applications in the gastrointestinal field comprise its use as, for instance, anti-diarrhoeals, inhibitors of gastro-oesophageal reflux and particularly antiemetics, e.g. in cancer patients receiving chemotherapy and radiation treatment. Further, serotonin is a potent broncho- and vasoconstrictor and thus the present antagonist may be used against hypertension and vascular disorders. In addition, serotonin antagonists have been associated with a number of other properties such as, the suppression of appetite and promotion of weight loss, which may prove effective in combating obesity; and also the alleviation of withdrawal symptoms in addicts trying to discontinue drinking and smoking habits.

Chemically, Paliperidone is the primary active metabolite of the older atypical antipsychotic Risperidone. Risperidone is extensively metabolized in the liver: the main metabolic pathway is through hydroxylation into 9-hydroxy Risperidone (Paliperidone), by the enzyme Cytochrome P450 2D6 (CYP 2D6). Paliperidone has a pharmacological profile and potency comparable with that of the parent drug Risperidone, but has a longer elimination half-life. Risperidone is distributed to and eliminated from the brain tissues more rapidly than its metabolite Paliperidone.

Paliperidone is a compound of formula (I) 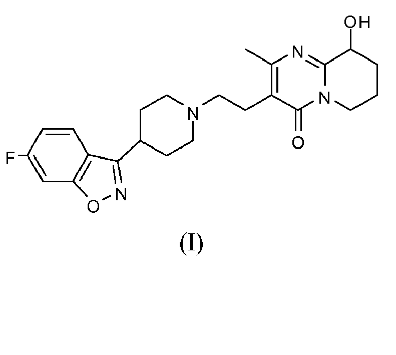 chemically known as 3-[2-[4-(6-fluoro-1,2-benzisoxazol-3-yl)-1-piperidinyl]ethyl]-6,7,8,9-tetrahydro-9-hydroxy-2-methyl-4*H*-pyrido[1,2-a]pyrimidin-4-one, which is disclosed in EP 368 388 B1 and marketed worldwide under the trade name Invega^{®}.

Like any synthetic compound, Paliperidone can contain extraneous compounds or impurities that can come from many sources. They can be unreacted starting materials, chemical derivatives of impurities contained in starting materials, by-products of the reaction, products of side reactions, or degradation products. Impurities in Paliperidone or any active pharmaceutical ingredient (API) are undesirable and, in extreme cases, might even be harmful to a patient being treated with a dosage form containing the API.

The purity of the API produced in the commercial manufacturing process is clearly a necessary condition for commercialization. Impurities introduced during commercial manufacturing processes must be limited to very small amounts, and are preferably substantially absent. The International Conference on Harmonization of Technical Requirements for Registration for Human Use ("ICH") guidance for API manufacturers requires that process impurities be maintained below set limits by specifying the quality of raw materials, controlling process parameters, such as temperature, pressure, time, and stoichiometric ratios, and including purification steps, such as crystallization, distillation, and liquid-liquid extraction, in the manufacturing process.

The product mixture of a chemical reaction is rarely a single compound with sufficient purity to comply with pharmaceutical standards. Side products and by-products of the reaction and adjunct reagents used in the reaction will, in most cases, also be present in the product mixture. At certain stages during processing of the API, Paliperidone, it must be analyzed for purity, typically, by HPLC, TLC or GC analysis, to determine if it is suitable for continued processing and, ultimately, for use in a pharmaceutical product. Purity standards are set with the intention of ensuring that an API is as free of impurities as possible, and, thus, are as safe as possible for clinical use. As discussed above, in the United States, the Food and Drug Administration guidelines recommend that the amounts of some impurities be limited to less than 0.1 percent.

Paliperidone is nearly insoluble in all organic solvents, which makes its purification quite difficult. Very few processes have been disclosed till now for the purification of Paliperidone.

EP 368388-B1, in example 3, the crude Paliperidone obtained is purified by column chromatography and the resulting residue is crystallized from acetone and recrystallized from isopropanol.

Patent applications WO2008/021346 and WO2008/140641 disclose several crystallizations of crude Paliperidone in an organic solvent, by solving it in a solvent and precipiting it, by mixing with an anti-solvent, by slurrying it in an organic solvent or by admixing its solution with a finely powdered of carbon and filtering to enhance the purity of Paliperidone, that would contain less than 0.1% of known or unknown impurities.

US 20090048272 A1 provides processes for the purification of Paliperidone providing a solution of Paliperidone in a suitable solvent or mixture of solvents, cooling the solution, and recovering the solid formed.

WO2009/060297 relates to a process for the preparation of pure Paliperidone by making its acid addition salts.

WO2009/11607 discloses a process for isolation Paliperidone pure from water with acid-base purification.

Paliperidone prepared by the processes known up till now can only be obtained in a satisfactory quality after running through a number of process steps or successive crystallizations, which lead to poor yields and use of large quantities of solvent.

### Summary of the invention

It has now been found a process for the preparation of pure Paliperidone in a straightforward manner.

An aspect of the present invention is to provide an economical and industrial process for the purification of Paliperidone.

The present invention provides a process for purifying Paliperidone comprising submitting crude Paliperidone, or a hydrate, a solvate, or a polymorph thereof, to an esterification reaction to yield a compound of formula (III) 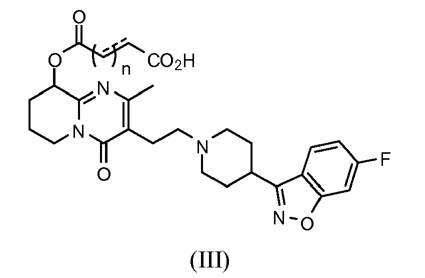 wherein n is 1-8, in presence of a basic agent.

The process of the present invention for purifying Paliperidone, further comprises the hydrolysis of the Paliperidone ester of formula (III), as defined above, to afford purified Paliperidone.

In another aspect, the present invention provides the compound of formula (III), as defined above, or a pharmaceutically acceptable salt, or a hydrate, a solvate, a polymorph thereof.

The present invention also provides the use of a compound of formula (III), or a pharmaceutically acceptable salt, hydrate, solvate or polymorph thereof, as intermediate in the preparation of Paliperidone or a pharmaceutically acceptable salt thereof.

The present invention also provides a pharmaceutical composition comprising Paliperidone purified with the process according to the present invention, optionally together with at least one pharmaceutically acceptable excipient.

The present invention also provides the use of Paliperidone purified with the process according to the present invention, for the preparation of a medicament.

### Description of figures

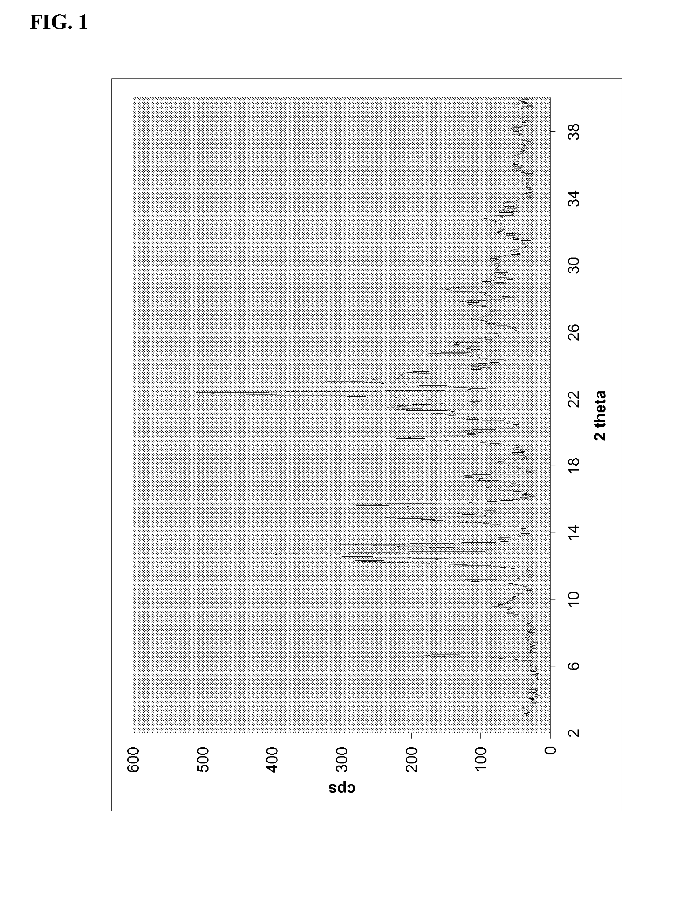

FIG. 1 provides an XRPD pattern of the compound of formula (IIIa).

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The term "alkyl" refers to a straight or branched hydrocarbon having from 1 to 8 carbon atoms. Examples of alkyl groups include, without limitation, methyl, ethyl, *n*-propyl, isopropyl, *n*-butyl, *sec*-butyl, isobutyl, *tert*-butyl, pentyl, hexyl, heptyl, octyl, and the like.

The term "alkenyl" refers to a straight or branched hydrocarbon having from 1 to 8 carbon atoms, which contain one or more double bonds. Examples of alkenyl groups include, without limitation, ethenyl, propenyl butenyl, vinyl, allyl, cyclohexenyl, 1,4-butadienyl, and the like.

The term "aryl" refers to a radical derived from one of the known ring systems with 1-4 rings, wherein each one of the rings forming said ring system:
has 3-7 carbon atoms,
is saturated, partially unsaturated or aromatic, and
is partially/totally fused.

According to the present invention when the ring system is formed by "isolated" rings means that the ring system is formed by two, three or four rings and said rings are bound via a bond from the atom of one ring to the atom of the other ring. The term "isolated" also embraces the embodiment in which the ring system has only one ring. Illustrative non-limitative examples of known ring systems consisting of one ring are those derived from: cyclopropyl, cyclobutyl, cyclopentyl, cyclhexyl, cycloheptyl, cyclopropenyl, cyclobutenyl, cyclopentenyl, phenyl, and cycloheptenyl.

According to the present invention when the ring system has rings "totally fused", means that the ring system is formed by two, three or four rings in which two or more atoms are common to two adjoining rings. Illustrative non-limitative examples are 1,2,3,4-tetrahydronaphthyl, 1-naphthyl, 2-naphthyl, anthryl, or phenanthryl,

According to the present invention when the ring system is "partially fused" it means that the ring system is formed by three or four rings, being at least two of said rings totally fused (i.e. two or more atoms being common to the two adjoining rings) and the remaining ring(s) being bound via a bond from the atom of one ring to the atom of one of the fused rings.

The term "pharmaceutically acceptable salt(s)", as used herein, unless otherwise indicated, includes salts of basic groups which may be present in the compounds of the present invention. The compounds prepared according to the present invention that are basic in nature are capable of forming a wide variety of salts with various inorganic and organic acids. The acids that may be used to prepare pharmaceutically acceptable acid addition salts of such basic compounds are those that form non-toxic acid addition salts i.e., salts containing pharmacologically acceptable anions, such as the hydrochloride, hydrobromide, hydroiodide, nitrate, sulfate, bisulfate, phosphate, acid phosphate, isonicotinate, acetate, lactate, salicylate, citrate, acid citrate, oxalate, tartrate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p-toluenesulfonate and pamoate [i.e., 1,1'-methylene-bis-(2-hydroxy-3-naphthoate)] salts. The compounds prepared according to the present invention may form pharmaceutically acceptable salts with various amino acids, in addition to the acids mentioned above.

Salts may be prepared according to processes well known in the art. One may prepare a pharmaceutically acceptable base salt by contacting Paliperidone with a sufficient amount of a desired acid to produce a nontoxic salt. If the salt precipitates from solution, it may be isolated by filtration; otherwise, the salt may be recovered, for instance, by evaporating the solvent.

The term "solvate" refers to a molecular complex comprising Paliperidone and a stoichiometric or non-stoichiometric amount of one or more solvent molecules (e.g., ethanol).

The term "hydrate" refers to a solvate comprising Paliperidone and a stoichiometric or non-stoichiometric amount of water.

The term "polymorph" refers to substances that have the same chemical formula than Paliperidone, but different crystal structures.

The term "crude" refers to Paliperidone containing different extraneous compounds, side products, by-products, and adjunct reagents (collectively "impurities") that can come from many sources. The impurities that can be present in a crude sample can include unreacted starting materials, chemical derivatives of impurities contained in starting materials, by-products of the reaction, products of side reactions, or degradation products. The impurities can also include solvents that are present or trapped in the crude compound. Crude Paliperidone does not meet the necessary condition for commercialization, for clinical use and the ICH guidelines recommended levels of impurities within specific limits, i.e. any single impurity below 0.1% and total amount of impurities below 0.5%. Generally, impurities are identified spectroscopically and/or with another physical method, and then associated with a peak position, such as that in a chromatogram, or a spot on a TLC plate (Strobel p. 953, Strobel, H.A.; Heineman, W.R., Chemical Instrumentation: A Systematic Approach, 3rd dd. - Wiley & Sons: New York 1989).

The term "pure Paliperidone" refers to Paliperidone containing less than about 0.1% of each single impurity. Preferably, the Paliperidone of the present invention contains less than about 0.05% and more preferably less than about 0.02% of each single impurity. The purity is preferably measured by HPLC, and is presented as % area as shown in the HPLC chromatogram. The possible impurities in Paliperidone include risperidone, raw materials, intermediates and other process-related impurities. The most significant impurity in the Paliperidone purified according the present invention is risperidone. Advantageously the process of this invention is able to provide Paliperidone with less than 0.1% of this impurity and of any single impurity.

The pure Paliperidone of the present invention has a total purity of at least about 98%. Preferably, the total purity is at least about 99%, most preferably at least about 99.9%. For example, the total purity of the pure Paliperidone of the present invention can be about 98% to about 99.95%, about 98% to about 99.99%, about 99% to about 99.95%, or about 99% to about 99.99%.

The term "excipient" herein means any substance, not itself a therapeutic agent, used as a carrier or vehicle for delivery of a therapeutic agent to a subject, or added to a pharmaceutical composition, to improve its handling or storage properties or to permit or facilitate formation of a dose unit of the composition into a discrete article such as a tablet, capsule, pill, powder, granule, pellet, lozenge, pastille, elixir, syrup, solution, suspension, emulsion, drop, lotion, spray, tincture, cream, ointment, gel, unguent, suppository and transdermal devices for oral, enteral, parenteral or topical administrations.

The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient.

The term "about" encompasses the range of experimental error that may typically occur in a measurement.

Certain formulae may also include one "-----" to indicate that the chain can contain at least one double bond.

It is contemplated that the purified Paliperidone following the process of the present invention as well as the compounds of formula (III) can be found or isolated in the form of hydrates or solvates, in different polymorphic forms, i.e., different crystalline forms, which are considered to fall within the scope of the present invention.

Generally, the chemical transformations described throughout the specification may be carried out using substantially stoichiometric amounts of reactants, though certain reactions may benefit from using an excess of one or more of the reactants. Additionally, many of the reactions disclosed throughout the specification, may be carried out at room temperature, but particular reactions may require the use of higher or lower temperatures, depending on reaction kinetics, yields, and the like. Furthermore, many of the chemical transformations may employ one or more compatible solvents, which may influence the reaction rate and yield. Depending on the nature of the reactants, the one or more solvents may be polar protic solvents, polar aprotic solvents, non-polar solvents, or some combination.

The process of the present invention is illustrated in the following Scheme 1, wherein the esterification reaction is carried out by reacting crude Paliperidone with a compound of formula (II) 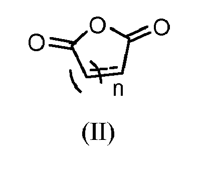 wherein n is 1-8;
or with a compound of formula (IV), 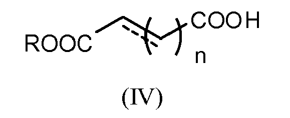 wherein R is a C₁-C₈ alkyl, C₁-C₈ alkenyl or aryl group, and n is as defined above. The compound of formula (III) is then hydrolysed to give the Paliperidone (I) in purified form.

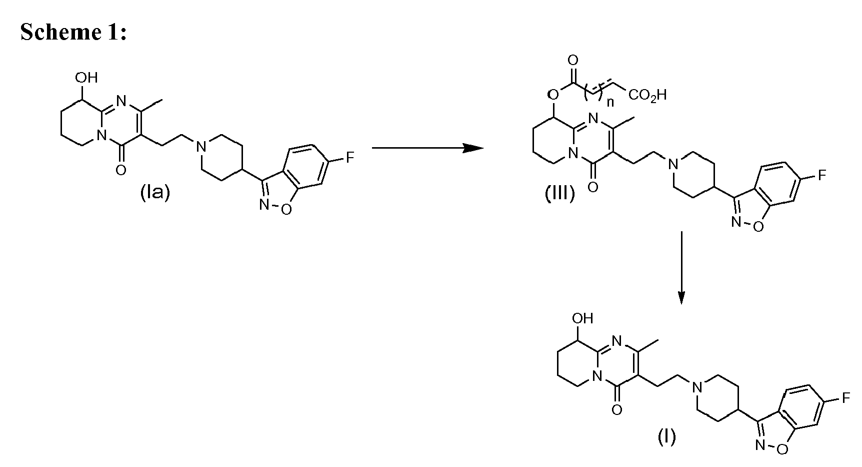

Crude Paliperidone (Ia), prepared as described in EP 368.388 B1 or a hydrate, a solvate, a polymorph thereof, is converted in an ester of formula (III), as defined above, by reacting it with a compound of formula (II) or of formula (IV), as defined above, in the presence of a basic agent.

Preferably, crude Paliperidone is converted in an ester of formula (III), by reacting the crude Paliperidone with a compound of formula (II), wherein n is 1-8, preferably n is 1-6, and more preferably n is 1-2. Preferred compounds of formula (II) are selected from the group consisting of: succinic anhydride, (compound of formula (II) wherein n = 1); maleic anhydride, (compound of formula (II) wherein n = 1 and one double bond is present); and glutaric anhydride, (compound of formula (II) wherein n = 2).

Examples of suitable basic agents include inorganic or organic bases. Preferably, the inorganic base can be alkali metal hydroxydes or alkoxydes, such as, sodium hydroxide, potassium hydroxide, sodium methoxide, sodium ethoxide, potassium t-butoxide, and the like. Preferably, the organic bases are pyridine, triethylamine, diisopropylamine, diisopropylethylamine, 4-dimethylaminopyridine, 1,8-diazabicyclo[5,4,0]undec-7-ene, and the like. A preferred basic agent of the present invention is diisopropylethylamine. The esterification may be conducted in any suitable solvent from room temperature to reflux. A suitable solvent of the present invention is a solvent in which the starting material can be dissolved in suitable conditions for the reaction to be done. A preferred solvent is an halogenated solvent, more preferably dichloromethane.

The ester intermediate of formula (III) can be purified very easily by employing a conventional method well known to those skilled in the art, such as extraction, crystallization, column chromatography, washing with an organic solvent or with an aqueous solution, eventually adjusting pH, or by transforming it into a salt.

Compounds of formula (III) are novel compounds and are a further object of the present invention. Preferred compounds of formula (III) are selected from the group consisting of:
4-(3-(2-(4-(6-fluorobenzo[d]isoxazol-3-yl)piperidin-1-yl)ethyl)-2-methyl-4-oxo-6,7,8,9-tetrahydro-4*H*-pyrido[1,2-a]pyrimidin-9-yloxy)-4-oxobutanoic acid (IIIa);
5-(3-(2-(4-(6-fluorobenzo[d]isoxazol-3-yl)piperidin-1-yl)ethyl)-2-methyl-4-oxo-6,7,8,9-tetrahydro-4*H*-pyrido[1,2-a]pyrimidin-9-yloxy)-5-oxopentanoic acid (IIIb); and
4-(3-(2-(4-(6-fluorobenzo[d]isoxazol-3-yl)piperidin-1-yl)ethyl)-2-methyl-4-oxo-6,7,8,9-tetrahydro-4*H*-pyrido[1,2-a]pyrimidin-9-yloxy)-4-oxobut-2-enoic acid.

In another aspect, the present invention provides the use of compounds of formula (III) as intermediates in the preparation of Paliperidone or a salt pharmaceutically acceptable salt thereof.

Pure Paliperidone (I), displaying a final level of each single impurity below 0.1%, as recommended by the ICH guidelines, may be obtained by hydrolysis of compounds of formula (III). The procedure of the hydrolysis of the ester of formula (III) to the corresponding alcohol derivative is well known to those of ordinary skill in the art. Preferably the hydrolysis of the ester of formula (III) to the corresponding alcohol derivative is obtained by action of a basic agent in a suitable solvent. Basic agents include, but are not limited to, inorganic bases, such as alkali metal hydroxides, for example sodium or potassium hydroxide; alkali metal alkoxides, such as sodium methoxide, sodium ethoxide, potassium tert-butoxide; alkali metal carbonates, such as sodium or potassium carbonate. Preferred bases are sodium hydroxide and potassium hydroxide.

Any suitable solvent known to those skilled in the art can be used for the hydrolysis of the ester of formula (III). Suitable solvents are polar protic solvents, such as water, an alcohol, such as methanol, ethanol, and the like; polar aprotic solvents, such as a ketone, for example acetone; an ester, for example ethyl acetate; a nitrile, for example acetonitrile; apolar solvents, such as an halogenated hydrocarbon, for example dichloromethane; an ether, for example diethyl ether, or a mixture of them. Preferably, the solvent is a mixture of acetone/dichloromethane.

The process of the present invention is suitable for the purification of Paliperidone on an industrial scale and the product obtained by process of the invention displays a purity about 99.9%.

Paliperidone obtained with the process of the present invention can be converted according to methods well known in the art into a pharmaceutically acceptable salt or converting the salt thereof into the free Paliperidone. One may regenerate the free base by contacting the acid addition salt with a base. Though certain physical properties of the free base and its respective acid addition salt may differ (e.g., solubility, crystal structure, hygroscopicity, etc.), a compound's free base and acid addition salt are otherwise the same for purposes of this disclosure. Paliperidone, obtained with the process according to the present invention, may be used for the preparation of a medicament.

The present invention also provides a pharmaceutical composition comprising Paliperidone obtained with the process according to the present invention optionally together with at least one pharmaceutically acceptable excipient. Excipients include, by way of illustration and not limitation, diluents, fillers, agglutinants, disintegrants, disintegration inhibitors, absorption accelerators, binders, carriers, suspensing/dispersing agents, film formers/coatings, adhesives, antiadherents, wetting agents, lubricants, glidants, preservatives, sorbents, surface active agents, substances added to mask or counteract a disagreeable taste or odor, flavorings, colorants, fragrances, aromatising agents, sweeteners and substances added to improve appearance of the composition. A person skilled in the art is aware of a whole variety of such excipient compounds suitable to formulate a pharmaceutical composition. The choice of excipients will to a large extent depend on factors such as the particular mode of administration, the effect of the excipient on solubility and stability, and the nature of the dosage form.

While the present invention has been described in terms of its specific embodiments, certain modifications and equivalents will be apparent to those skilled in the art and are included within the scope of the present invention.

In the following, the present invention shall be illustrated by means of some examples, which are not construed to be viewed as limiting the scope of the invention.

### Examples

The following abbreviations refer respectively to the definitions below:
DCM (dichloromethane); HPLC (High Performance Liquid Chromatography); MeOH (methanol); TLC (Thin Layer Chromatography); XRPD (X-Ray Powder Diffraction).
XRPD spectra were performed by means of an APD 2000 Ital Structures diffractometer at 25°C, using a CuKα tube (40 kV, 30 mA, λ = 1.5406 Å) as the X-ray source. Data collection was made in 2θ step scan mode and in Bragg-Brentano configuration, at a scan speed of 0.02°/s in the range of 3° to 40° in 2θ. The samples were placed on an aluminium sampler.

### Example 1

4-(3-(2-(4-(6-fluorobenzo[d]isoxazol-3-yl)piperidin-1-yl)ethyl)-2-methyl-4-oxo-6,7,8,9-tetrahydro-4*H*-pyrido[1,2-a]pyrimidin-9-yloxy)-4-oxobutanoic acid (IIIa) 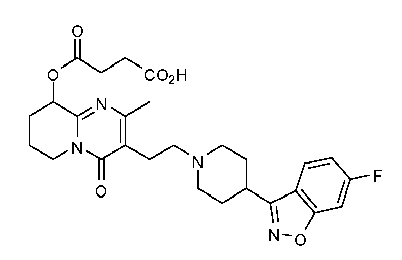 A 250 mL four necked round bottomed flask, fitted with reflux condenser, thermometer, rubber septum, mechanical stirrer and connected to a nitrogen manifold, was charged with crude Paliperidone (I) (containing approximately 0.5% of Risperidone as impurity by HPLC) (15.0 g, 35.17 mmol), 4-dimethylaminopyridine (197 mg, 1.61 mmol), dichloromethane (150 mL), diisopropylethylamine (7.2 mL, 42.21 mmol) and succinic anhydride (3.87 g, 38.69 mmol). The heterogeneous mixture was stirred at reflux for 16 hours checking the progress of the reaction by TLC (DCM-MeOH 9:1 + 1% aq. NH₄OH, UV visualization at 254 nm). The reaction mixture turned gradually to a solution.

The reaction was quenched by addition of water (100 mL) followed by 1N HCl (40 mL approximately) until pH= 6. The heterogeneous mixture was stirred for 15 minutes, the solid was filtered washing with DCM and acetone to obtain the product as a pink solid (16.7 g, Yield: 90%). The product can be crystallized from methanol affording a beige solid, showing the XRPD pattern reported in FIG. 1, comprising peaks at about 6.6; 11.1; 12.3; 12.7; 13.3; 14.9; 15.6; 19.6; 21.4; 22.3; 23.0° +/- 0.2° 2θ. Yield: 95%.

¹H NMR (300 MHz, *d₆*-dmso, 298K): δ 1.70-2.15 (m, 5H), 2.15-2.35 (m, 3H), 2.40-2.60 (m, 7H), 2.60-2.70 (m, 2H), 3.08-3.17 (m, 3H), 3.67-3.74 (m, 1H), 3.90-3.98 (m, 1H) 5.67 (t, *J*=5.2 Hz, 1H), 7.27 (td, ³*J*_{HF}=9.2, ⁴*J*_{HH}=1.8 Hz, 1H), 7.67 (dd, ³*J*_{HF} =9.2, ⁴*J*_{HH}=2.2 Hz, 1H), 8.00 (dd, ³*J*_{HH} =8.9, ⁴*J*_{HF}=5.5 Hz, 1H)

¹³C NMR (75 MHz, *d₆*-dmso, 298K): δ 17.4, 21.1, 23.2, 24.8, 28.9, 29.1, 29.8, 33.3, 33.8, 42.2, 52.7, 55.9, 68.8, 97.3 (d, ²*J*_{CF}=27.1 Hz), 112.6 (d, ²*J*_{CF}=25.2 Hz), 117.3, 120.1,

123.8 (d, ³*J*_{CF}=10.3 Hz), 152.7, 158.0, 161.3, 163.1 (d, ²*J*_{CF}=13.7 Hz), 163.5 (d, ¹*J*_{CF}=250.0 Hz), 171.2, 173.4

### Example 2

5-(3-(2-(4-(6-fluorobenzo[d]isoxazol-3-yl)piperidin-1-yl)ethyl)-2-methyl-4-oxo-6,7,8,9-tetrahydro-4*H*-pyrido[1,2-a]pyrimidin-9-yloxy)-5-oxopentanoic acid (IIIb)

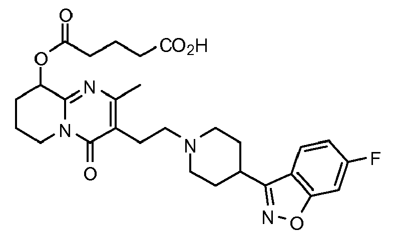

The title compound has been prepared according to Example 1, by reaction with glutaric anhydride (instead of succinic anhydride).

### Example 3

4-(3-(2-(4-(6-fluorobenzo[d]isoxazol-3-yl)piperidin-1-yl)ethyl)-2-methyl-4-oxo-6,7,8,9-tetrahydro-4*H*-pyrido[1,2-a]pyrimidin-9-yloxy)-4-oxobut-2-enoic acid (IIIc)

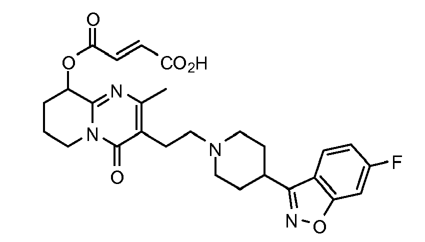

The title compound has been prepared according to Example 1, by reaction with maleic anhydride (instead of succinic anhydride).

### Example 4: Hydrolysis of compound (IIIa) in order to obtain the purified Paliperidone

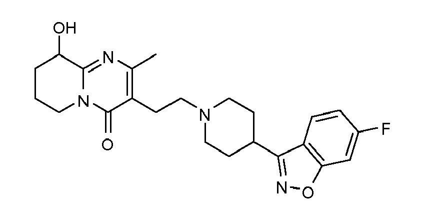

A 500 mL round bottomed flask was charged with Paliperidone hemisuccinate ester (IIIa) (15.0 g, 28.5 mmol), dichloromethane (200 mL), acetone (10 mL), water (150 mL) and sodium hydroxide (2.50 g, 62,6 mmol). The biphasic mixture was stirred at room temperature for 1 hour. The phases were separated and the organic phase was washed with saturated brine and concentrated to a residue to afford purified Paliperidone (11.2 g, Yield: 92%) containing less than 0.1 % Risperidone impurity by HPLC.

### Example 5: HPLC Assay and related Substances

### a) Analytical Method

Instrument: Waters Alliance e2695 Liquid Chromatograph, equipped with a variable
volume automatic injector and a UV-Visible Detector 2489;
Column: XBridge C18 4,6X75mm 2.5µm or equivalent
Flow rate: 1 mL/min
Injection volume: 10 µL
Wavelenght: 238 nm
Column temperature: 50°C
Sample tray: 25°C
Diluting solution: Water mQ/Acetonitrile (1:1)
Mobile phase:

### Phase A: Buffer 1.4 g of K₂HPO₄ in 1000 mL of water. Adjust pH to 3.0 with 5% H₃PO₄.

### Phase B: Acetonitrile/Water mQ (98:2)

Gradient:

**TABLE 1**

| Time (min) | %A | %B | Flow (ml/min) |
|---|---|---|---|
| 0 | 90 | 10 | 1.0 |
| 25 | 30 | 70 | 1.0 |
| 30 | 30 | 70 | 1.0 |
| 31 | 90 | 10 | 1.0 |

Equilibration time: 9 minutes
Run time: 30 minutes
Analysis time: 40 minutes

### b) Determination of impurities in crude Paliperidone.

Following the method (a), it was determined the impurities present in crude Paliperidone by HPLC. The results are shown in TABLE 2:

**TABLE 2: Impurities in Paliperidone crude**

| | Name | Retention Time | Relative Retention time | %Area |
|---|---|---|---|---|
| 1 | N-oxid | 11.305 | 0.89 | **0.79231** |
| 2 | | 11.891 | 0.94 | 0.00588 |
| 3 | Paliperidone | 12.668 | 1.00 | **92.32085** |
| 4 | Risperidone | 14.215 | 1.12 | **0.67863** |
| 5 | | 14.487 | 1.14 | 0.16089 |
| 6 | | 15.063 | 1.19 | 0.01325 |
| 7 | | 15.553 | 1.23 | 0.01928 |
| 8 | | 16.981 | 1.34 | 0.11576 |
| 9 | | 17.599 | 1.39 | 0.26992 |
| 10 | | 18.271 | 1.44 | 0.11178 |
| 11 | | 18.615 | 1.47 | 0.26996 |
| 12 | | 19.197 | 1.52 | 0.08757 |
| 13 | | 19.674 | 1.55 | 0.01700 |
| 14 | | 20.047 | 1.58 | 0.04136 |
| 15 | | 20.368 | 1.61 | 0.03123 |
| 16 | | 21.023 | 1.66 | 1.26447 |
| 17 | | 21.880 | 1.73 | 3.27294 |
| 18 | | 22.527 | 1.78 | 0.52690 |

### c) Determination of the impurities in Paliperidone purified from dimethylacetamide (state of the art)

The purification process was the following:
A 2 L round bottomed flask was charged with crude Paliperidone (320 g, 92.3 % purity by HPLC at λ=238 nm) and N,N dimethylacetamide (800 mL). The suspension was heated to 100-105 °C until complete dissolution of the product, then the mixture was cooled to 8-10 °C in 2 hours, the product was filtered and washed with acetone. The procedure was repeated twice more. After drying under vacuum at 70 °C until constant weight the product (280 g) was analyzed by HPLC. The analytical results are shown in TABLE 3 below.

**TABLE 3: Impurities in Paliperidone purified from dimethylacetamide**

| | Name | Retention time (min) | Relative Retention time | % Area |
|---|---|---|---|---|
| 1 | N-Oxid | 11.291 | 0.89 | **0.19868** |
| 2 | Paliperidone | 12.646 | 1.00 | **97.06384** |
| 3 | Risperidone | 14.189 | 1.12 | **0.52111** |
| 4 | | 16.958 | 1.34 | 0.05182 |
| 5 | | 17.542 | 1.39 | 0.02600 |
| 6 | | 19.225 | 1.52 | 0.01801 |
| 7 | | 19.995 | 1.58 | 0.03192 |
| 8 | | 20.333 | 1.61 | 0.05389 |
| 9 | | 20.695 | 1.64 | 0.10814 |
| 10 | | 20.987 | 1.66 | 0.45227 |
| 11 | | 21.546 | 1.70 | 0.24768 |
| 12 | | 21.850 | 1.73 | 0.65289 |
| 13 | | 22.496 | 1.78 | 0.36717 |
| 14 | | 23.327 | 1.84 | 0.20658 |

### d) Determination of the impurities in the purified Paliperidone resulting following the process of the invention (Example 4).

The resulting purified Paliperidone of Example 4 was also analyzed by HPLC. The results are shown in TABLE 4.

**TABLE 4: Impurities in Paliperidone purified following the process of the invention**

| | Name | Retention Time (min) | Relative Retention Time | % Area |
|---|---|---|---|---|
| 1 | N-oxid | 10.795 | 0.89 | **0.08221** |
| 2 | Paliperidone | 12.164 | 1.00 | **99.85447** |
| 3 | Risperidone | 13.800 | 1.13 | |
| 4 | | 14.764 | 1.21 | 0.05705 |
| 5 | | 20.084 | 1.66 | 0.00627 |

Surprisingly, carrying out the purification process of the invention almost all the impurities present in the Paliperidone crude are eliminated (as it can be seen comparing the data of TABLES 2 and 4). The impurities eliminated from the Paliperidone crude (following the process of the invention) are those having the following relative retention times: 0.94, 1.12, 1.19, 1.23, 1.34, 1.39, 1.44, 1.47, 1.52, 1.55, 1.58, 1.61, 1.73, and 1.78. Additionally, impurities identified as those having the relative retention times 0.89 and 1.66 are significantly reduced when the Paliperidone is purified following the process of the invention.

It is clear from the comparison of TABLE 3 and TABLE 4, that the process of the invention is more effective in the purification of Paliperidone: it is achieved a highly pure Paliperidone (with about 99.8% of purity) with only three impurities which represent less than 0.2% over the total amount of the product.

The data shown in TABLES 2-4 show, therefore, that the process of the invention comprising the step of esterifying a compound of formula (III) in the presence of a basic solvent and its ulterior hydrolysis, provides an efficient way of obtaining a highly pure Paliperidone, practically free of all the impurities present in the starting crude.

### Bibliographic references

1. EP 368388
2. WO 2008021346
3. WO 2008140641
4. US 20090048272
5. WO 2009060297
6. WO 2009116071
7. Strobel, H.A.; Heineman, W.R., Chemical Instrumentation: A Systematic Approach, p. 953, - Wiley & Sons: New York 1989 3rd dd. - Wiley & Sons: New York 1989.

## Claims

1. A process for purifying Paliperidone comprising submitting crude Paliperidone to an esterification reaction to yield a compound of formula (III) 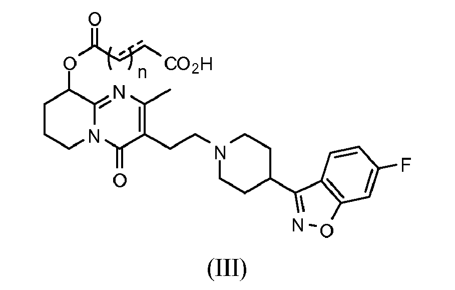 wherein n is 1-8,
in presence of a basic agent.

2. The process according to claim 1, wherein the esterification reaction is carried out by reacting the crude Paliperidone with a compound of formula (II) 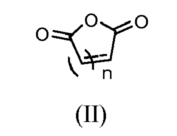 wherein n is 1-8;
or with a compound of formula (IV), 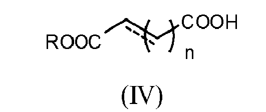 wherein R is an C₁-C₈ alkyl, C₁-₈ alkenyl or aryl group and n is as defined above.

3. The process according to claim 2, wherein the esterification reaction is carried out with the compound of formula (II).

4. The process according to claim 3, wherein the compound of formula (II) is selected from the group consisting of: succinic anhydride, glutaric anhydride, and maleic anhydride.

5. The process according to claim 1, wherein the basic agent is an organic base; preferably diisopropylethylamine.

6. The process according to claim 1, wherein the esterification reaction is conducted in an halogenated solvent, preferably dichloromethane.

7. The process according to claim 1, further comprising the step of hydrolysis of the ester of formula (III) to afford purified Paliperidone.

8. The process according to claim 7, wherein the hydrolysis of the ester of formula (III) is carried out in the presence of a basic agent, preferably an inorganic base, more preferably sodium hydroxide or potassium hydroxide.

9. The process according to claim 7, wherein the hydrolysis of the ester of formula (III) is carried out in a mixture of acetone/dichloromethane.

10. The process according to any one of the claims 7-9, further comprising converting the purified Paliperidone into a pharmaceutically acceptable salt thereof.

11. A compound of formula (III), or a pharmaceutically acceptable salt thereof 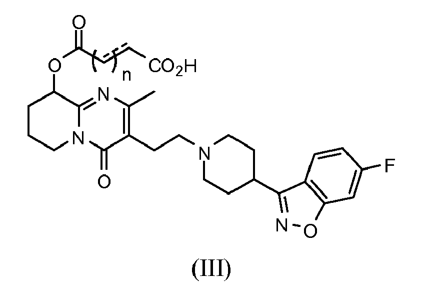 wherein n is 1-8.

12. The compound according to claim 11, wherein n is 1-6, preferably n is 1-2.

13. The compound according to any one of the claims 11-12, which is selected from the group consisting of:
4-(3-(2-(4-(6-fluorobenzo[d]isoxazol-3-yl)piperidin-1-yl)ethyl)-2-methyl-4-oxo-6,7,8,9-tetrahydro-4*H*-pyrido[1,2-a]pyrimidin-9-yloxy)-4-oxobutanoic acid;
5-(3-(2-(4-(6-fluorobenzo[d]isoxazol-3-yl)piperidin-1-yl)ethyl)-2-methyl-4-oxo-6,7,8,9-tetrahydro-4*H*-pyrido[1,2-a]pyrimidin-9-yloxy)-5-oxopentanoic acid; and
4-(3-(2-(4-(6-fluorobenzo[d]isoxazol-3-yl)piperidin-1-yl)ethyl)-2-methyl-4-oxo-6,7,8,9-tetrahydro-4*H*-pyrido[1,2-a]pyrimidin-9-yloxy)-4-oxobut-2-enoic acid.

14. Use of the compound of formula (III) as defined in claim 11, or a pharmaceutically acceptable salt thereof, as intermediate in the preparation of Paliperidone or a pharmaceutically acceptable salt thereof.
